# EUROPEAN PATENT APPLICATION

(11) **EP 1 939 289 A1**
(43) Date of publication of application: **02.07.2008**
(21) Application number: 06090225.1
(22) Date of filing: 29.12.2006
(51) Int. Cl.: C12N 15/11, C07K 14/47

(54) **Polynucleotide sequence for the inhibition of phospholamban synthesis**

(71) Applicant: Charité-Universitätsmedizin Berlin, 10117 Berlin (DE); Freie Universität Berlin, 14195 Berlin (DE)
(72) Inventor: Fechner, Henry, Dr., 15926 Luckau (DE); Kurreck, Jens, Dr., 12205 Berlin (DE); Prömel, Simone, 12159 Berlin (DE)
(74) Representative: Gross, Felix

(57) **Abstract**

A DNA polynucleotide sequence is disclosed that comprises a transcribed sequence, and a promoter sequence. Transcription of the transcribed sequence by an RNA polymerase in a cell renders an RNA a transcript capable of forming a partially self-complementary hairpin structure which can be processed by the cell to an siRNA product that can degrade phospholamban mRNA in such cell. The promoter is a drug inducible conditional promoter or a cardiomyocyte cell-specific promoter or both, operably linked to the transcribed sequence. The promoter is operable by an RNA polymerase naturally occurring in a mammalian cell.

## Description

This invention relates to reagents that facilitate the inhibition of phospholamban in heart tissue of higher animals. More specifically, the invention relates to polynucleotide sequences capable of down-regulating phospholamban expression in a patient's cell, as specified in the independent claims.

Cardiac insufficiency and congestive heart failure are pathologies of major importance. They are associated with deficiencies in cardiac contractility and abnormalities in intracellular calcium handling.

Phospholamban (PLB) is a 52 amino acid integral membrane protein that takes part in the regulation of the calcium ion pump in muscle cells. In heart muscle cells, PLB controls the sarco-endoplasmatic reticulum Ca²⁺ ATPase pump (SERCA). The PLB/SERCA ratio influences cardiac contractility. Increased expression of SERCA and inhibition of PLB both work towards improved contractility in failing cardiomyocytes. The mRNA (cds) of phospholamban is published in the National Center of Biotechnology Information (NCBI) nucleotide database as NM_002667.2.

It has been shown that over-expressing SERCA can restore contractility, calcium handling and frequency response in isolated cardiomyocytes (Del Monte et al., Circulation 1999, 100:2308-2311 ).

Different approaches are possible for down-regulating the PLB activity in a cell. US2004121942A1, for example, employs expression of exogenous mutated PLB protein to attain this effect. Another approach is the use of ribonucleic acid (RNA) such as antisense or siRNA to suppress gene expression at the level of mRNA control. Since RNA is not a stable molecule that lends itself easily to pharmaceutical formulation and use, direct application of RNA molecules is not necessarily the most promising clinical approach to apply RNA to the management of human disease. One approach circumventing RNA instability is the use of chemically modified ribonucleotides, such as thiophosphate backbone molecules or 2'deoxy purine or 2'-O-methly pyrimidine moieties. Comparable approaches during the antisense era have suffered from toxicity issues caused by the molecules and their degradation products.

An alternative approach that avoids application of RNA molecules is to transcribe RNA within the patient's cell from deoxyribonucleic acid (DNA) expression cassettes. DNA is more stable under many conditions encountered in drug delivery, and different technologies are known to deliver DNA expression cassettes into the cells of patients. These include viral transfer, particle-mediated bombardment of cells, injection of naked DNA or DNA complexed by polycations or liposomes into tissue, and other methods known in the context of so-called "gene therapy".

It has been attempted to restore contractility of failing heart muscle cells *in-vitro* by inhibiting PLB expression with antisense RNA molecules expressed from adenoviral vectors in cultured cardiomyocytes (Eizema et al., Circulation 2000; 101; 2193-2199; and Del Monte et al., Circulation 2002; 105; 904-907).

Different enzymes transcribe RNA from DNA in a eukaryotic cell, specifically, RNA polymerase I, II and III. Transcription from each polymerase is controlled by different regulatory elements regulating the expression of the associated gene. Traditionally, expression of short, non-mRNA molecules from expression cassettes artificially introduced into cells have made use of RNA polymerase III (Pol-III). Pol-III synthesizes 5S ribosomal and tRNA. The activity of RNA Pol-III is not controlled in a cell-specific fashion. US2005064489 describes Pol-III promoters regulated by the tetracycline transactivator system. Such conditionally active promoters offer the advantage of being able to control the state of activity of the artificially introduced transgene in a patient; they do not, however, offer cell specificity if applied to the entire body of a patient by systemic delivery.

In any clinical approach that attempts to regulate gene expression, cell or tissue specificity is a highly desirable element of control and safety. Hence, means of facilitating PLB-targeted RNA expression from cell-specific promoters such as RNA polymerase II promoters for the control of phospholamban expression in heart cells would be advantageous.

Antisense regulation of PLB from Pol-II promoters has been attempted by expressing antisense RNA under the control of the CMV or the inducible atrial natriuretic factor (ANF) promoter (Eizema et al., Circulation 2000; 101; 2193-2199).

Attempts to down-regulate expression of intracellular proteins by antisense approaches have generally not achieved the clinical successes expected from the technology at the time of its inception. A new approach to down-regulate intracellular gene expression is the use of so-called small interfering RNA (siRNA) molecules. These are pairs of short, double-stranded RNA molecules typically 19-29 base pairs in length, one strand of which is complementary to a section of mRNA. The targeted mRNA is degraded by the RISC complex. siRNA molecules were originally discovered in plants (Hamilton and Baulcombe, Science. 1999 Oct 29;286(5441 ):886), and later mammalian cells (Elbashir et al., Nature. 2001 May 24;411 (6836):428-9, WO0244321).

Closely related is the discovery of microRNA or miRNA. These are RNA molecules naturally synthesized from genomically encoded genes that, upon processing by cellular RNA-modifying activities, render short RNA hairpin structures with 11 bp (basepair) stem structures. miRNA also targets mRNA for degradation and is probably a mechanism of gene expression control within the cell.

US26198825A1 shows siRNA sequences that are suggested to target phospholamban synthesis. The document also mentions the results of an *in-vitro* experiment, whereby PLB-mRNA is reduced in cultured cardiomyocytes by siRNA from a commercial kit in a non-dose-responsive manner.

One approach to the generation of siRNA in-vivo is the generation of so-called small hairpin RNA (shRNA), which is a natural precursor of siRNA. Such shRNA molecules can be generated by expression of a partially self-complementary RNA molecule from an expression cassette introduced into a eukaryotic cell. The technology is reviewed by McIntyre and Fanning (BMC Biotechnol. 2006; 6:1).

Additional technical obstacles exist to express shRNA from Pol-II promoters, recently led to the development of so-called microRNA-generating vectors that can be used to drive siRNA-generation from a Pol-11-promoter (Shin et al., Proc. Nat. Acad. USA 103, 13759-13764). This technology has recently been made available by Invitrogen Inc. as the pcDNA 6.2-GW/miR plasmid.

In view of the cited state of the art, one objective of the invention is to provide an RNA molecule that can lead to the down-regulation of phosholamban expression in a cell of the heart, or an expression cassette from which such RNA molecule can be transcribed.

Another objective of the invention is to provide a reagent that can be applied to a patient suffering from a condition associated with the activity of phospholamban, or a condition which could benefit from reduction of phospholamban expression, where the reagent would, upon application to a patient, lead to the down-regulation of phospholamban synthesis in heart cells of the patient.

These objectives are attained by the invention as it is specified in the independent claims.

Specifically, according to one aspect of the invention, an RNA molecule is provided that is capable of forming a partially self-complementary hairpin structure which can be processed by a mammalian cell to an siRNA product capable of degrading a phospholamban mRNA in that cell. "Self-complementary" in this context signifies that the RNA molecule has two sequence tracts that allow canonical base-pairing in 5' to 3' direction of one sequence tract with the other in reverse orientation, so that the RNA linear polymer can fold back on itself to produce a RNA double-helical structure. Such hairpin RNA molecule, formed for example from a transcript of DNA-dependent RNA polymerase in a cell from a transgene, may be processed by intracellular RNA-degrading activities to yield a functional siRNA molecule capable of down-regulating the expression of the mRNA it is targeted to, in case of this invention, phospholamban mRNA.

The hairpin structure can also be defined as one of its hybridizing strands being highly similar to a sequence comprised in the phospholamban mRNA sequence, the GeneBank reference for which is given above. Sequence similarity quantification is well known in the art of molecular biology; computational tools for quantification of sequence similarity are found, among other places, at the EMBL-EBI website at http://www.ebi.ac.uk/Tools/similarity.html. According to the invention, the hybridizing sequences of the RNA molecule can be 80%, 85%, 90%, 95%, 97% or 100% similar to a sequence tract in phospholamban mRNA, for example the coding sequence of phospholamban mRNA; the 5' untranslated region or the 3' untranslated region.

According to another aspect of the invention, an expression cassette for the intracellular expression of a phospholamban-specific inhibitory RNA is provided, which can be processed by the cell in which it is expressed, to down-regulate the expression of phospholamban synthesis by that cell. Such expression cassette comprises a promoter sequence operable in a mammalian cell, a transcription initiation sequence operable by a mammalian RNA polymerase, and a transcribed sequence, also referred to as a first sequence element.

The transcribed sequence comprises a DNA sequence, which, when transcribed by an RNA polymerase in a cell, renders a transcript capable of forming a partially self-complementary hairpin structure that can be processed by the cell to yield an siRNA product. Such siRNA product typically is a 19 to 29 base pair long RNA double strand with short overhangs on the 3' end, capable of degrading an mRNA in said cell. According to the invention, the hairpin structure and the resulting siRNA product is at least partially complementary to a section of the phospholamban mRNA sequence synthesized in that cell.

Specifically, according to one aspect of the invention, a transcribed sequence leads to the transcription of a small-hairpin RNA (shRNA) molecule that can be processed by the cell to yield a siRNA molecule targeting PLB-mRNA. Preferably, such transcribed sequence is transcribed either under the control of a promoter operable by RNA polymerase III, or under the control of a promoter operable by RNA polymerase II (Pol-II).

In the context of this specification, "a promoter being operable by an RNA polymerase" means that the presence of the promoter sequence characterized as "a promoter" is known to lead to recruitment of the necessary cellular factors and the recruitment and initiation of transcription of said RNA polymerase. "Operably linked" in the context of this specification means that a promoter sequence and a transcribed sequence are linked in such way that the promoter will, under normal physiological conditions, lead to the transcription of the transcribed sequence. Hence, a "Pol-II-promoter" under normal physiological conditions is able to recruit RNA polymerase II to the transcribed sequence operably linked thereto, and lead to the initiation of transcription of that transcribed sequence.

If the promoter is a Pol-II promoter, the transcribed sequence preferably comprises additional sequence elements that facilitate the processing of the initial Pol-II-derived transcript by the splicing apparatus in the cell, to yield a mature shRNA molecule that is able to target PLB-mRNA and to down-regulate the expression of PLB in a cell in which the expression cassette is transcribed.

Examples of shRNA sequences that have been employed in the context of the present invention are SEQ ID NO 001, SEQ ID NO 002 and SEQ ID NO 003. The bases no. 6 to 26 of these sequences comprise, when transcribed into RNA, the part of the small hairpin RNA that will later form the mature siRNA directed against the target mRNA. These sequences are underscored below, with their respective complementary part on the 3' end in boldface:

misi-hPL1 229 (SEQ ID NO 001): TGCTGTGTTG AGGCATTTCA ATGGTTGTTT TGGCCACTGA
CTGACAACCA TTGATGCCTC AACA

misi-hPL2 301 (SEQ ID NO 002): TGCTGATACA GATCAGCAAG AGACATGTTT TGGCCACTGA
CTGACATGTC TCTCTGATCT GTAT

misi-hPL3 214 (SEQ ID NO 003): TGCTGATGGT TGAGGCTCTT CTTATAGTTT TGGCCACTGA
CTGACTATAA GAAGCCTCAA CCAT

Sequences are known that serve as signals to direct the processing of RNA molecules by the splicing apparatus of the cell. Splicing is catalyzed by the spliceosome, which is a large RNA-protein complex composed of five small nuclear ribonucleoproteins present in eukaryotic cells. Examples for additional sequence elements that facilitate the processing of the initial Pol-II-derived transcript by the processing and splicing apparatus in the cell to yield a mature shRNA molecule are splice donor and splice acceptor sites. Examples for additional sequence elements that facilitate the processing of the initial Pol-II-derived transcript, and which have successfully been employed in the context of the present invention, are the 5' miR flanking region (SEQ ID NO 004) and the 3' miR flanking region (SEQ ID NO 005). Transcribed sequences that comprise a shRNA-generating element and splice donor and acceptor sites for liberation of that transcribed element are referred to as misiRNA (from micro-siRNA) in the context of this invention.

SEQ ID NO 004: ctggaggctt gctgaaggct gtatgctg

SEQ ID NO 005: aggacacaag gcctgttact agcactcaca tggaacaaat ggccc

A transcribed sequence comprising a transcription initiation sequence for Pol-II and the transcribed region (the + strand) including splice donor and acceptor sites and the shRNA element are shown in SEQ ID NO 006.

According to one aspect of the invention, transcription of the transcribed sequence can be effected by a RNA-polymerase II (Pol-II) promoter. One such promoter is the Cytomegalovirus immediate early promoter, commonly referred to as CMV promoter. Other commonly employed promoters are the simian virus 40 (SV40) promoter, Rous Sarcoma Virus (RSV) promoter and EF1alpha promoter. According to one aspect of the invention, a Pol-II promoter is preferred that is specific for cardiomyocytes. "Specific" in the context of the present invention means that an expression cassette comprising the promoter that is specific for cardiomyocytes and the first sequence element operably linked thereto, is transcribed in cardiomyocytes at a significantly higher rate than in other cell types. An example for a cardiomyocyte-specific promoter are the sequence MLC1500 (SEQ ID No 007) MLC800 (SEQ ID No 008) (Muller et al. 2006, Cardiovascular research, 70, 70-78.) and MLC260 (SEQ ID No 009).

According to another aspect of the invention, a conditional Pol-II promoter is preferred that can be induced in the patient, preferably by administration of a small molecule pharmaceutical compound. One example for such conditional promoter is the tetracycline transactivator system reviewed by Gossen and Bujard (Ann. Rev. Genet. 2002, 36:153-73). In this "tet-on" system, gene expression is regulated by presence of tetracycline or doxycycline by binding of the tetracycline transactivator protein to tetracycline response elements on the DNA. A conditional promoter in the context of the present specification shall signify a promoter the activity of which can be significantly increased by modifying directly or indirectly a parameter extrinsic to the cell in which the promoter is active. Nonlimiting examples for such conditional promoters are promoters with different activities depending on the concentration of a small molecule drug, or depending on the temperature of the cell, or the ionic strength of a specific ion in the cell or its surrounding.

A sequence comprising the conditional promoter of the tet-on-system and a transcribed sequence yielding a misiPLB-shRNA molecule and comprising the TRE-tight1 promoter construct (Sipo et al. 2006, JMM, 84:215-225) is shown in SEQ ID NO 010. Therein, bases no. 1-252 comprise the TetO7 site, bases 253 -314 are the CMV minimal promoter sequence with base 306 (thymine) being the putative transcriptional start site, and from base 394 to 453 the misiPLBh-shRNA flanked by the 3' miR flanking region, the 5' miR flanking region and the SV40 polyadenylation sequence.

Similarly, a TRE-tight2 promoter as described by Sipo et al. (as cited above) can be employed.

According to another aspect of the invention, the expression cassette is administered to a patient in form of a viral vector. Different viral vectors can be employed for gene transfer into a patient's cells. Retroviruses, especially lentiviruses have been employed. Lentivirus-mediated gene transfer can lead to stable integration of a transgene into a non-dividing cell. Herpesvirus family members have also been employed for gene transfer.

According to one aspect of the invention, an adenovirus is used to mediate the transfer of the expression cassette for the expression of an RNA molecule for PLB down-regulation in a cell. Certain adenovirus subtypes are known to exhibit tropism for heart muscle cells and can thus be employed to transfer the inventive expression cassette into cardiomyocytes. Adenovirus type 5 is one example for a virus exhibiting heart tissue tropism.

Methods and experimental protocols for producing adenovirus virions comprising an expression cassette for therapeutic or vaccination purposes are well known in the art (for a current review, see Bangari and Mittal, Curr Gene Ther. 2006 Apr;6(2):215-26). Adenovirus vectors can be produced by transfection of an adenoviral DNA into a helper cell line (e.g HEK 293) which expresses adenoviral proteins (e.g. E1A, E1B), that can not be expressed from the adenoviral genome as the respective genes were deleted from the genome. An adenoviral vector in the context of the present specification thus means a DNA sequence comprising an expression cassette as specified above, comprising the following elements:
- a promoter operable in a mammalian myocardial cell,
- a transcribed first sequence element comprising a shRNA-generating sequence that upon transcription will render a RNA transcript, which can be processed by the cellular RNA processing apparatus into an siRNA directed against PLB-mRNA, and
- the virus-derived sequences necessary to effect packaging of the DNA sequence into virion particles by a suitable packaging cell line for production of replication-defective adenovirus virions containing the expression cassette.

According to another aspect of the invention, an adeno-associated virus (AAV) is used to mediate the transfer of the expression cassette for the expression of an RNA molecule for down-regulation of PLB in a cell. AAV are non-pathogenic and lead to little if any immune reaction against them. Expression of AAV-borne expression transgenes is stable over a long time period.

Certain AAV subtypes are known to exhibit tropism for heart muscle cells and can thus be employed to transfer the inventive expression cassette into cardiomyocytes. AAV9 is one example for a virus exhibiting heart tissue tropism.

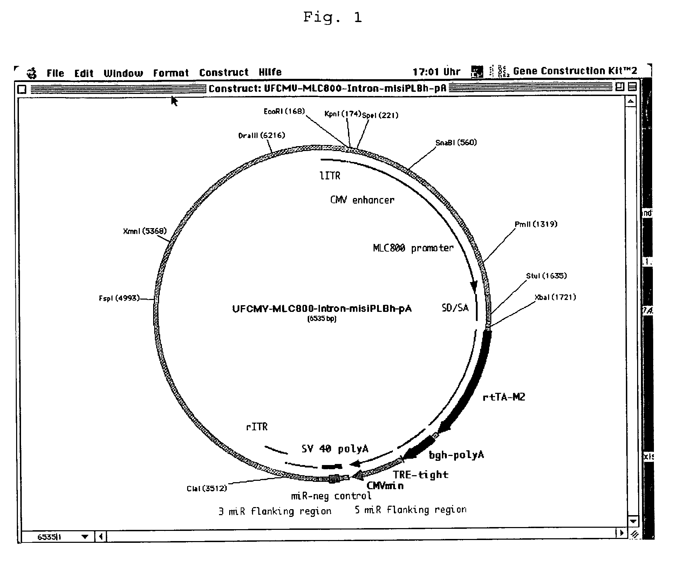

AAV9 vectors are produced by cotransfection methods. The vector genome comprises the ITR (inverted terminal repeat) sequences of AAV2 or ITR sequences of other AAVs adjacent to the expression cassette for the expression of the transgenes (Plasmid: UFCMV-MLC800-Intron-misiPLBh-pA). The AAV genome is packaged into an AAV9 capsid (Plasmid: p5E18-VD2/9; contains AAV-Rep2 gene + CAP9 gene from AAV, a kind gift from Dr. Wilson, University of Pennsylvania, USA). Further proteins essential for packaging are derived from Plasmid 3 (e.g. E1A, VA-RNA of adenovirus). AAV vector particles are produced in cultured cells and released from cell culture by three freeze/thaw cycles and caesium gradient centrifugation (Grimm: Methods (2002), 28, 146-157). One example of an AAV vector genome containing tetracycline regulated misiPLBshRNA expression cassette is shown in SEQ ID NO 011 (for the vector chart, see Fig. 1).

AAV can be produced as single stranded or self complementary (sc) AAV vectors. A scAAV vector contains a small mutation in one terminal resolution site, making it possible to package a dimeric vector genome. One example for a scAAV vector comprising an inventive misi-RNA construct able to down-regulate phospholamban mRNA is shown in SEQ ID NO 012 (scAAV-CMV800 misiPLBh)

According to one preferred embodiment, an expression cassette comprising a misiRNA for the generation of a shRNA molecule targeting PLB expression, under the control of a tet-on conditional promoter system and comprising the packaging sequence elements for packaging the expression cassette into an AAV vector is shown in SEQ ID NO 011.

According to yet another aspect of the present invention, an adeno-associated-virus virion comprising the expression cassette of the previous paragraph, is provided. Preferably, this virion is a AAV type 9 (AAV9) virion. Similarly, a composition for the treatment of cardiomyopathy comprising an expression cassette as disclosed in the previous paragraph, or a virion as disclosed in this paragraph, is provided. Such virion may be obtained by co-transfecting a suitable expression cassette such as the expression cassette of the previous paragraph or specifically, an expression cassette as exemplified by SEQ ID NO 011, into a suitable cell line, along with a plasmid containing the elements for packaging the expression cassette into AAV9 virions.

Methods and experimental protocols for producing adeno-assovciated virus virions comprising an expression cassette for therapeutic or vaccination purposes are well known in the art. (Grimm, Methods, 28, 146-157). An AAV vector in the context of the present specification thus means a DNA sequence comprising an expression cassette as specified above, comprising a promoter operable in a mammalian myocardial cell, and a transcribed sequence comprising a shRNA-generating RNA transcript able to render an siRNA directed against PLB-mRNA, and the adeno-associated virus-derived sequences necessary to effect packaging of the DNA sequence into a suitable packaging cell line for production of AAV viral particles containing the expression cassette.

According to yet another aspect of the present invention, an improved conditional promoter is provided for the use in an expression cassette controlling the transcription of short hairpin RNA that can be processed into siRNA in the cell. The tet-on system currently in use consists of a CMV promoter driving the expression of a recombinant tetracycline transactivator protein (rtTA), which, in turn, binds to down-stream tetracycline response elements (TRE) on the same DNA strand, leading to expression of a transcribed sequence under control of the TRE. In theory, transcription of the transcribed sequence only proceeds when tetracycline is present. The system, however, suffers, in our experience, from constitutive expression in the absence of the inducing drug, probably because the very strong CMV promoter "reads through" the entire downstream sequence and produces a transcript that includes the downstream shRNA-generating sequence, thus leading to shRNA production from the CMV promoter instead of the drug-controlled TRE elements. On the other hand the leakiness of the TRE element may be inhibited by replacement of the minimal CMV promoter through another non leaky promoter as tight2 (see above for sequence details), but also by use of an tetracycline-controlled tanscriptional silencer (tTS) which binds to the TetO7 and represses the CMVmin (Fechner et al. 2003, Gene Therapy, 2003, 10, 1680-1690).

According to the invention, this problem can be circumvented by exchanging the CMV promoter driving the expression of the rtTA protein, for a cell-specific, less active promoter such as MLC260 (SEQ ID NO 009)

According to another preferred embodiment, an expression cassette comprising a misiRNA for the generation of a shRNA molecule targeting PLB expression, under the control of a cardiomyocyte-specific promoter in an AAV packaging vector is provided.

An AAV vector in the context of the present specification is the preferred embodiment of the invention. It comprises a DNA sequence a polynucleotide sequence as specified above, comprising the following elements:
- a promoter operable in a mammalian myocardial cell, preferably a myocard-specific promoter, more preferably a myocard-specific conditional promoter such as the tet-on system under control of the MLC260 promoter,
- a transcribed first sequence element comprising a shRNA-generating sequence that upon transcription will render a RNA transcript, which can be processed by the cellular RNA processing apparatus into an siRNA directed against PLB-mRNA, and
- the virus-derived sequences necessary to effect packaging of the DNA sequence into virion particles by a suitable packaging cell line for production of replication-defective AAV virions, preferably AAV9 virions, containing the expression cassette.

Alternatively, according to yet another aspect of the invention, the expression cassette for the expression of an RNA molecule for down-regulation of PLB in a cell can be applied to the patient as a naked DNA or as DNA in association with a non-viral transfer agent, for example encapsulated in liposomes or in association with polyamine reagents such as polyethylenimine. Expression cassettes either in association with a virus, with a nonviral transfer agent or as naked DNA can be applied by intravenous injection, intramuscular injection, particle-mediated gene transfer or any other suitable transfer method.

According to yet another aspect of the present invention, an expression construct expressing an inhibitory RNA construct such as described above, can be combined with an expression construct facilitating an increased expression of SERCA. Methods for expressing polypeptides in human cells are well known in the art, and include the methods of viral, liposomal and naked DNA transfer discussed above. mRNA can be expressed from SERCA coding sequences under control of Pol-II promoters such as CMV or inducible or cell-specific promoters, as discussed above. SERCA mRNA is published as GeneBank entry NM_170665.

According to the aforementioned aspect of the invention, an expression construct encoding SERCA may be located on the same polynucleotide sequence as the construct leading to phospholamban down-regulation. It may also be separated from the latter and packaged into the same or distinct virions, or associated into liposomal or cationic particles together with the phospholamban-specific shRNA generating construct.

Patients in the context of this invention can be humans, however it is apparent that the invention can also be applied to higher animals, such as dogs or horses as well as primates.

According to another aspect of the invention, the sequence of SEQ ID NO 009 can be used to drive transcription of any transgene, for example a therapeutic gene such as SERCA, an RNA construct or any other transcribed sequence, in a cardiac-cell-specific fashion.
- Fig. 1: shows a vector chart of a plasmid vector for packaging into AAV virions, containing an expression cassette comprising a cardiac cell-specific conditional (tet-on) promoter and a phosholamban mRNA targeting misiRNA construct. The sequence of the vector is given in SEQ ID NO 011.

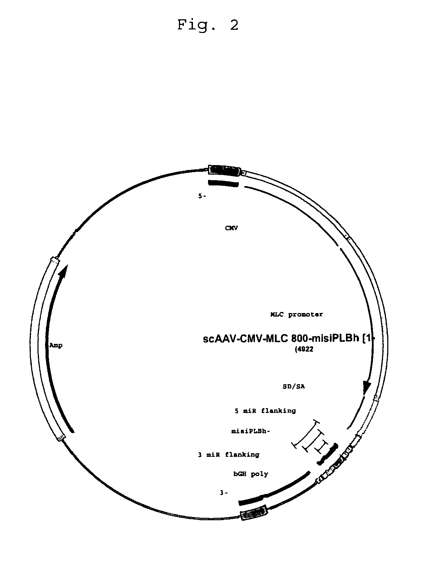

- Fig. 2: shows a vector chart of a plasmid vector for packaging into AAV virions, containing an expression cassette comprising a cardiac cell-specific promoter and a phosholamban mRNA targeting misiRNA construct. The sequence of the vector is given in SEQ ID NO 012.
- Fig.3: shows experimental results of shRNA expression against phospholamban mRNA in cells expressing a phospholamban-GFP-fusion construct. Specifically, Fig. 3 a and b show western blot results, Fig. 3 c shows the expression inhibition efficacy as ratios of the densitometric evaluation of the western blots.

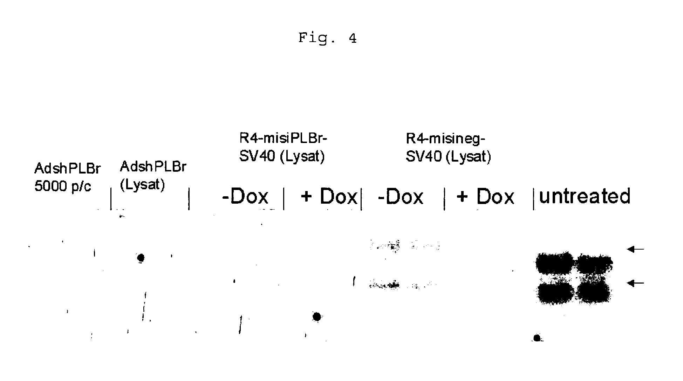

- Fig. 4: shows Northern blots with radioactive RNA transcripts blotted against PLB-mRNA from myocardiac cells (see Example 2). Lane 1-4: positive control, Adenovirus anti-PLB shRNA transcribed from an U6 promoter as published previously (Fechner et al. 2006 Gene Therapy Highly efficient and specific modulation of cardiac calcium homeostastis by adenovector-derived short hairpin RNA targeting PLB. DEU: 10.1038); lane 1,2: neonatal rat cardiomyocytes infected with caesium chloride gradient purified AdV particles expressing rat PLB-shRNA from an U6 (polymerase III promoter), lane 3, 4: neonatal rat cardiomyocytes infected with an HEK 293T cell lysate containing AdV particles expressing rat PLB-shRNA from an polymerase III promoter; lane 5-8 : neonatal rat cardiomyocytes infected with an HEK 293T cell lysate AdV particles expressing rat misiPLB-shRNA with and without doxycyclin, lane 9-12: negative control - neonatal rat cardiomyocytes infected with an HEK 293T cell lysate AdV particles expressing scambled-shRNA with and without doxycyclin, lane 13,14: neonatal rat cardiomyocytes (untreated) construct with and without doxocyclin, lane 9-12 : negative control (vector without misiRNA construct).

### Examples

### Example 1: misiRNA expression down-regulates expression of human PLB in cos7 cells.

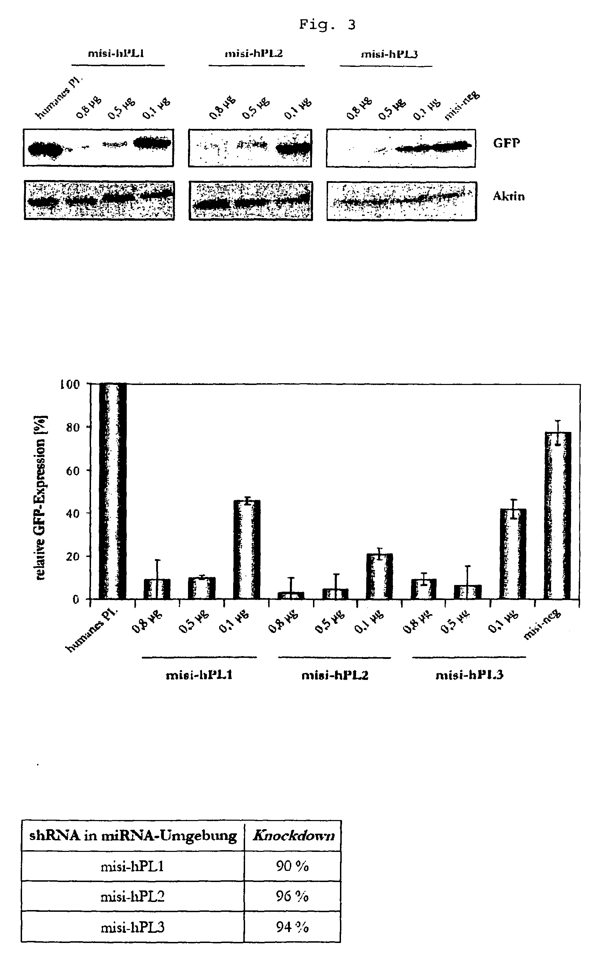

Cos7 cells were co-transfected with a plasmid expressing a GFP - human PLB fusion transcript (Fechner et al. 2006 Gene Therapy Highly efficient and specific modulation of cardiac calcium homeostastis by adenovector-derived short hairpin RNA targeting PLB. DEU: 10.1038). Cells were harvested 48 h later and analyzed by Western-blot analysis for GFP expression. All human misiPLB-shRNA exhibited down-regulated GFP expression in a dose-dependent manner, indicating that the expression of the fusion construct was silenced by the misiPLB-shRNA construct. See Fig 3.

### Example 2: Adenovirus mediated PLB knock-down by expression of misiPLB-shRNA

Neonatal rat cardiomyocytes were transduced with the respective adenoviral vectors or cell lysate from HEK293 containing adenoviral vector particles for 2h, medium was replaced and fresh medium added. Total RNA was isolated 4 days later and investigated for PLB-mRNA expression by Northern-blotting. Results (Northern-blot) are given in Fig. 4: Adenovirus vectors expressing rat PLB-shRNAs strongly suppressed PLB-mRNA expression. Similar silencing efficiency was found with the misiPLBr-shRNA expression vector R4-misiPLBr-SV40. However, PLB silencing was seen in the presence and absence of Doxycyclin (Dox), indicating probable leakiness or polymerase run-through from the upstream CMV promoter. Adenoviral vectors expressing a control misi-RNA (R4-misineg-SV40) was used as control.

Sequence Protocol

shRNA-generating DNA Sequence for the Inhibition of Phospholamban
Synthesis

SEQ ID NO 001 misi-hPL1 229

S: TGCTGTGTTGAGGCATTTCAATGGTTGTTTTGGCCACTGACTGACAACCATTGATGCCTCAACA

SEQ ID NO 002 misi-hPL2 301 S:
TGCTGATACAGATCAGCAAGAGACATGTTTTGGCCACTGACTGACATGTCTCTCTGATCTGTAT

SEQ ID NO 003 misi-hPL3 214 S:
TGCTGATGGTTGAGGCTCTTCTTATAGTTTTGGCCACTGACTGACTATAAGAAGCCTCAACCAT

SEQ ID NO 004

5' miR flanking region

ctggaggctt gctgaaggct gtatgctg

SEQ ID NO 005

3' miR flanking region

aggacacaag gcctgttact agcactcaca tggaacaaat ggccc

SEQ ID NO 006

A transcribed sequence comprising a transcription initiation
sequence for Pol-II and the transcribed region (the + strand)
including splice donor and acceptor sites and the shRNA element are
shown in SEQ ID NO 006

Cagatcgcct ggagaattcg agctcggtac cccggaggta gtgagtcgac cagtggatcc
tggaggcttg ctgaaggctg tatgctgata cagatcagca agagacatgt tttggccact
gactgacatg tctctctgat ctgtatcagg acacaaggcc tgttactagc actcacatgg
aacaaatggc ccagatctgg ccgcatcgag atcgacgcgt gctagaggat cataatcagc
cataccacat ttgtagaggt tttacttgct ttaaaaaacc tcccacacct ccccctgaac
ctgaaacata aaatgaatgc aattgttgtt gttaacttgt ttattgcagc ttataatggt
tacaaataaa gcaatagcat cacaaatttc acaaataaag catttttttc actgcctcta gc

SEQ ID NO 007

MLC1500-Promoter

GGTACCGCGGTGGCGGCCGCTTCGAGCTCGCCCGACATTGATTATTGACTAGTTATTAATAGTAATCA
ATTACGGGGTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCATAGTAACGC
CAATAGGGACTTTCCATTGACGTCAATGGGTGGAGTATTTACGGTAAACTGCCCACTTGGCAGTACAT
CAAGTGTATCATATGCCAAGTACGCCCCCTATTGACGTCAATGACGGTAAATGGCCCGCCTGGCATTA
TGCCCAGTACATGACCTTATGGGACTTTCCTACTTGGCAGTACATCTACGTATTAGTCATCGCTATTA
CCATGGTGATGCGGTTTTGGCAGTACATCAATGGGCGTGGATAGCGGTTTGACTCACGGGGATTTCCA
AGTCTCCACCCCATTGACGTCAATGGGAGTTTGTTTTGGCACCAAAATCAACGGGACTTTCCAAAATG
TCGTAACAACTCCGCCCCATGCGGCCGCTCTAGCTAGCCTTGAACTCACTATGTAGGCAAGCATGACC
ATGAACTTCTGATCCTCCTTCCTCAGTGTCCTGGGATAACAGGTGTGTGTCACTCCCTACCCTTCTAA
TAGCAATATGTGGCCACATGTTTGTGCCCCACAGGTTGAGACCATCTTGACCTGAGGAAGAAATAGCT
AACACTCACCTCCTGAAGGTTGCCTGGATCTCGTCTTTGTCTTTCCAGCACTCAGGAGTGGGGGGGTC
AGAAGTGCAAAGTCAGCCCCTGCTACATAATGAGTTCAAGGCTCGCCTGGGCTACATGAGACCATGCC
TCAAAAAGAAAAGGAATTGGTATAGTGACATACTCTGGTCCTCCCAGTACTTAGGGACACAGAGGCCA
CTCCACCACCATCTCCAGCAGCTGGCCTGCCTCCCCGAGCCTCGTTTATTTCATATCAATGAGATGGG
GACCCAACTGCTAAGGTGACCTTGCACCCACGGGGTGACTGGAGACTTGAGAGTGGAGGGTTTATCAT
TTCTCCAGTCGGTCAGCAAGTGGTCGCCGCCAAGAAGGTTTTGAGTTCAAAGTAGAAGATGGGACAGG
GAGAGACCAGCGAGAAGACCCCACCCTGGAGCTGACTGTCCCTGTGCGGCTGGGTGGGGACACAAAGC

AGAGAAGCAGAGGCAGAGAACAAGGGTGGGTGACATTTGAGCAAGGATGGGGGTGTGCCAGAGGCTGC
CCAAGATGCATAGGTGCAAAGGCCCTGAGGTTCGAGGATGCCTGGATCCGGAATCAAAGCTCAGGCTC
CTCCCTCTTCCTCCTCCTCCTCTGCCCCCTCCTCCTCCTCTGCCCCCTCTTCCTCCTCTGCCCCCTCT
TCTTCCTCCTCCTCTTCCTCCTCCCCTCCTCATCTACCTCCTTCTCCTCCTCCTCCCCCTCCTCTTCC
TCCTCTGCCCCCTCTTCCTCCTCCTCCTCTTCCTCCTCCTCTTCCTCCTCCCCTCCTCATCTACCTCC
TTCTCCTCCTCCTCCCCCTCCTCTTCCTCCTCTGCCCCCTCTTCCTCCTCTGCCCCTCTTCCTCCTCC
TCCTCTTCCTCCTCTGCCCCCTCCTCCCCCTCCTCTTCCTCTTCCTCCTCCCCTCCTCATCTACCTCC
TTCTCTTCCTCCTCTTCTTCCTCCTCTTTCTCCTCCTCCTCCCTCTCCTCTTCCTCCTCCTCTTCTTT
CTCCTCCTCCTCTTCCTCCCCCTCCCCTTCCTGGGTTACTTTTCCCCATTAGACAATGGCAGGACCCA
GAGCACAGAGCATCGTTCCCAGGCCAGGCCCCAGCCACTGTCTCTTTAACCTTGAAGGCATTTTTGGG
TCTCACGTGTCCACCCAGGCGGGTGTCGGACTTTGAACGGCTCTTACTTCAGAAGAACGGCATGGGGT
GGGGGGGCTTAGGTGGCCTCTGCCTCACCTACAACTGCCAAAAGTGGTCATGGGGTTATTTTTAACCC
CAGGGAAGAGGTATTTATTGTTCCACAGCAGGGGCCGGCCAGCAGGCTCCTTG

SEQ ID NO 008 MLC 800 Promoter

CGGTACCGCGGTGGCGGCCGCTTCGAGCTCGCCCGACATTGATTATTGACTAGTTATTAATAGTAATC
AATTACGGGGTCATTAGTTCATAGCCCATATATGGAGTTCCGCGTTACATAACTTACGGTAAATGGCC
CGCCTGGCTGACCGCCCAACGACCCCCGCCCATTGACGTCAATAATGACGTATGTTCCCATAGTAACG
CCAATAGGGACTTTCCATTGACGTCAATGGGTGGAGTATTTACGGTAAACTGCCCACTTGGCAGTACA
TCAAGTGTATCATATGCCAAGTACGCCCCCTATTGACGTCAATGACGGTAAATGGCCCGCCTGGCATT
ATGCCCAGTACATGACCTTATGGGACTTTCCTACTTGGCAGTACATCTACGTATTAGTCATCGCTATT
ACCATGGTGATGCGGTTTTGGCAGTACATCAATGGGCGTGGATAGCGGTTTGACTCACGGGGATTTCC
AAGTCTCCACCCCATTGACGTCAATGGGAGTTTGTTTTGGCACCAAAATCAACGGGACTTTCCAAAAT
GTCGTAACAACTCCGCCCCATGCGGCCGCTCTAGCCGGAATCAAAGCTCAGGCTCCTCCCTCTTCCTC
CTCCTCCTCTGCCCCCTCCTCCTCCTCTGCCCCCTCTTCCTCCTCTGCCCCCTCTTCTTCCTCCTCCT
CTTCCTCCTCCCCTCCTCATCTACCTCCTTCTCCTCCTCCTCCCCCTCCTCTTCCTCCTCTGCCCCCT
CTTCCTCCTCCTCCTCTTCCTCCTCCTCTTCCTCCTCCCCTCCTCATCTACCTCCTTCTCCTCCTCCT
CCCCCTCCTCTTCCTCCTCTGCCCCCTCTTCCTCCTCTGCCCCTCTTCCTCCTCCTCCTCTTCCTCCT
CTGCCCCCTCCTCCCCCTCCTCTTCCTCTTCCTCCTCCCCTCCTCATCTACCTCCTTCTCTTCCTCCT
CTTCTTCCTCCTCTTTCTCCTCCTCCTCCCTCTCCTCTTCCTCCTCCTCTTCTTTCTCCTCCTCCTCT
TCCTCCCCCTCCCCTTCCTGGGTTACTTTTCCCCATTAGACAATGGCAGGACCCAGAGCACAGAGCAT
CGTTCCCAGGCCAGGCCCCAGCCACTGTCTCTTTAACCTTGAAGGCATTTTTGGGTCTCACGTGTCCA
CCCAGGCGGGTGTCGGACTTTGAACGGCTCTTACTTCAGAAGAACGGCATGGGGTGGGGGGGCTTAGG
TGGCCTCTGCCTCACCTACAACTGCCAAAAGTGGTCATGGGGTTATTTTTAACCCCAGGGAAGAGGTA
TTTATTGTTCCACAGCAGGGGCCGGCCAGCAGGCTCCTT

SEQ ID NO 009 MLC 260 Promoter

GCGGCCGCTTCGAGCTCGCCCGACATTGATTATTGACTAGTTATTAATAGTAATCAATTACGGGGTCA
TTAGTTCATAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAATAGGGACTT
TCCATTGACGTCAATGGGTGGAGTATTTACGGTAAACTGCCCACTTGGCAGTACATCAAGTGTATCAT
ATGCCAAGTACGCCCCCTATTGACGTCAATGACGGTAAATGGCCCGCCTGGCATTATGCCCAGTACAT
GACCTTATGGGACTTTCCTACTTGGCAGTACATCTACGTATTAGTCATCGCTATTACCATGGTGATGC
GGTTTTGGCAGTACATCAATGGGCGTGGATAGCGGTTTGACTCACGGGGATTTCCAAGTCTCCACCCC
ATTGACGTCAATGGGAGTTTGTTTTGGCACCAAAATCAACGGGACTTTCCAAAATGTCGTAACAACTC
CGCCCCATGCGGCCGCTCTAGGACCCAGAGCACAGAGCATCGTTCCCAGGCCAGGCCCCAGCCACTGT
CTCTTTAACCTTGAAGGCATTTTTGGGTCTCACGTGTCCACCCAGGCGGGTGTCGGACTTTGAACGGC
TCTTACTTCAGAAGAACGGCATGGGGTGGGGGGGCTTAGGTGGCCTCTGCCTCACCTACAACTGCCAA
AAGTGGTCATGGGGTTATTTTTAACCCCAGGGAAGAGGTATTTATTGTTCCACAGCAGGGGCCGGCCA
GCAGGCTCCTT

SEQ ID NO 010

TRE-Tight1 Promotor Konstrukt (Sipo et al. 2006, JMM, 84:215-
225)

1-252: TetO7; 253 -314 CMVmin; (t) 306: putative transcriptional
start site; 394 - 453: misiPLBh-shRNA; undrlined, italics, 5'
miR flanking region; SV40 poly A

Gagtttactccctatcagtgatagagaacgtatgtcgagtttactccctatcagtgatagag
aacgatgtcgagtttactccctatcagtgatagagaacgtatgtcgagtttactccctatca
gtgatagagaacgtatgtcgagtttactccctatcagtgatagagaacgtatgtcgagttta
tccctatcagtgatagagaacgtatgtcgagtttactccctatcagtgatagagaacgtatg
tcgaggtaggcgtgtacggtgggaggcctatataagcagagctcgtttagtgaaccgt
cagatcgcctggagaattcgagctcggtaccccg
gaggtagtgagtcgaccagtggatcctggaggcttgctgaaggctgtatgctgatacagatc
agcaagagacatgttttggccactgactgacatgtctctctgatctgtatcaggacacaagg
cctgttactagcactcacatggaacaaatggcccagatctggccgcatcgagatcgacgcgt
gctagaggatcataatcagccataccacatttgtagaggttttacttgctttaaaaaacctc
ccacacctccccctgaacctgaaacataaaatgaatgcaattgttgttgttaacttgtttat
tgcagcttataatggttacaaataaagcaatagcatcacaaatttcacaaataaagcatttt
tttcactgcctctagc

SEQ ID NO 011

UFCMV-MLC800-Intron-misiPLBh-pA
AAV2 5'ITR     6405 - 162
CMV enhancer 186-738
MLC promoter 800 739-1504
SD/SA 1509-1686

rtTA-M2 1727-2482
bGH poly A 2523-2756
TREmod (TRE-Tight) 2787-3029
CMVmin (minimaler CMV promoter) 3030-3094
5'miR flanking region 3146-3173
misiPLB-shRNA 3174-3233
3'miR flanking region 3234-3278
SV40 poly A 3314-3509
AAV2 3'ITR 3529-3694

ggggggggggggggggggccactccctctctgcgcgctcgctcgctcactgaggccgggc
gaccaaaggtcgcccgacgcccgggctttgcccgggcggcctcagtgagcgagcgagcgcgc
agagagggagtggccaactccatcactaggggttcctagatctgaattcggtaccgcggtgg
cggccgcttcgagctcgcccgacattgattattgactagttattaatagtaatcaattacgg
ggtcattagttcatagcccatatatggagttccgcgttacataacttacggtaaatggcccg
cctggctgaccgcccaacgacccccgcccattgacgtcaataatgacgtatgttcccatagt
aacgccaatagggactttccattgacgtcaatgggtggagtatttacggtaaactgcccact
tggcagtacatcaagtgtatcatatgccaagtacgccccctattgacgtcaatgacggtaaa
tggcccgcctggcattatgcccagtacatgaccttatgggactttcctacttggcagtacat
ctacgtattagtcatcgctattaccatggtgatgcggttttggcagtacatcaatgggcgtg
gatagcggtttgactcacggggatttccaagtctccaccccattgacgtcaatgggagtttg
ttttggcaccaaaatcaacgggactttccaaaatgtcgtaacaactccgccccatgcggccg
ctctagccggaatcaaagctcaggctcctccctcttcctcctcctctgcccccctcctcc
tcctctgcccccctcttcctcctctgcccccctcttcttcctcctcctcttcctcctcccctcc
tcatctacctccttctcctcctcctcccccctcctcttcctcctctgcccccctcttcctcctc
ctcctcttcctcctcctcttcctcctcccctcctcatctacctccttctcctcctcctcccc
ctcctcttcctcctctgcccccctcttcctcctctgcccctcttcctcctcctcctcttcctc
ctctgcccccctcctcccccctcctcttcctcttcctcctcccctcctcatctacctccttctc
ttcctcctcttcttcctcctctttctcctcctcctccctctcctcttcctcctcctcttctt
tctcctcctcctcttcctcccccctccccttcctgggttacttttccccattagacaatggca
ggacccagagcacagagcatcgttcccaggccaggccccagccactgtctctttaaccttga
aggcattttgggtctcacgtgtccacccaggcgggtgtcggactttgaacggctcttactt
cagaagaacggcatggggtgggggggcttaggtggcctctgcctcacctacaactgccaaaa
gtggtcatggggttatttttaaccccagggaagaggtatttattgttccacagcaggggccg
gccagcaggctccttgaatttcgaggaactgaaaaaccagaaagttaactggtaagtttagt
ctttttgtcttttatttcaggtcccggatccggtggtggtgcaaatcaaagaactgctcctc
agtggatgttgcctttacttctaggcctgtacggaagtgttacttctgctctaaaagctgcg
gaattgtacccgcggccgcccccaattcgagctcgcccggggatcctctagaatgtcaagac
tggacaagagcaaagtcataaacggcgctctggaattactcaatggagtcggtatcgaaggc
ctgacgacaaggaaactcgctcaaaagctgggagttgagcagcctaccctgtactggcacgt
gaagaacaagcgggccctgctcgatgccctgccaatcgagatgctggacaggcatcataccc
acttctgccccctggaaggcgagtcatggcaagactttctgcggaacaacgccaagtcattc
cgctgtgctctcctctcacatcgcgacggggctaaagtgcatctcggcacccgcccaacaga
gaaacagtacgaaaccctggaaaatcagctcgcgttcctgtgtcagcaaggcttctccctgg
agaacgcactgtacgctctgtccgccgtgggccactttacactgggctgcgtattggaggaa
caggagcatcaagtagcaaaagaggaaagagagacacctaccaccgattctatgcccccact
tctgagacaagcaattgagctgttcgaccggcagggagccgaacctgccttccttttcggcc
tggaactaatcatatgtggcctggagaaacagctaaagtgcgaaagcggcgggccggccgac
gcccttgacgattttgacttagacatgctcccagccgatgcccttgacgactttgaccttga
tatgctgcctgctgacgctcttgacgattttgaccttgacatgctccccgggtaactaagta
aggatccgtcgacaagcttctgtgccttctagttgccagccatctgttgtttgcccctcccc
cgtgccttccttgaccctggaaggtgccactcccactgtcctttcctaataaaatgaggaaa
ttgcatcgcattgtctgagtaggtgtcattctattctggggggtggggtggggcaggacagc

aaggggaggattgggaagacaatagcaggcatgctggggatgcggtgggctctatggcttc
tgaggcggaaagaaccagctggggtctagtcgaggccctttcgtcttcacgagtttactc
cctatcagtgatagagaacgtatgtcgagtttactccctatcagtgatagagaacgatgtcg
agtttactccctatcagtgatagagaacgtatgtcgagtttactccctatcagtgatagaga
acgtatgtcgagtttactccctatcagtgatagagaacgtatgtcgagtttatccctatcag
tgatagagaacgtatgtcgagtttactccctatcagtgatagagaacgtatgtcgaggtagg
cgtgtacggtgggaggcctatataagcagagctcgtttagtgaaccgtcagatcgcctggag
aattcgagctcggtacccggaggtagtgagtcgaccagtggatcctggaggcttgctgaag
gctgtatgctggaaatgtactgcgcgtggagacgttttggccactgactgacgtctccacgc
agtacatttcaggacacaaggcctgttactagcactcacatggaacaaatggcccagatctg
gccgcatcgagatcgacgcgtgctagaggatcataatcagccataccacatttgtagaggtt
ttacttgctttaaaaaacctcccacacctccccctgaacctgaaacataaaatgaatgcaat
tgttgttgttaacttgtttattgcagcttataatggttacaaataaagcaatagcatcacaa
atttcacaaataaagcatttttttcactgcctctagcaaatcgataactggggagagatcta
ggaacccctagtgatggagttggccactccctctctgcgcgctcgctcgctcactgaggccg
cccgggcaaagcccgggcgtcgggcgacctttggtcgcccggcctcagtgagcgagcgagcg
cgcagagagggagtggcccccccccccccccccctgcagcccagctgcattaatgaatcg
gccaacgcgcggggagaggcggtttgcgtattgggcgctcttccgcttcctcgctcactgac
tcgctgcgctcggtcgttcggctgcggcgagcggtatcagctcactcaaaggcggtaatacg
gttatccacagaatcaggggataacgcaggaaagaacatgtgagcaaaaggccagcaaaagg
ccaggaaccgtaaaaaggccgcgttgctggcgtttttccataggctccgcccccctgacgag
catcacaaaaatcgacgctcaagtcagaggtggcgaaacccgacaggactataaagatacca
ggcgtttccccctggaagctccctcgtgcgctctcctgttccgaccctgccgcttaccggat
acctgtccgcctttctcccttcgggaagcgtggcgctttctcaatgctcacgctgtaggtat
ctcagttcggtgtaggtcgttcgctccaagctgggctgtgtgcacgaaccccccgttcagcc
cgaccgctgcgccttatccggtaactatcgtcttgagtccaacccggtaagacacgacttat
cgccactggcagcagccactggtaacaggattagcagagcgaggtatgtaggcggtgctaca
gagttcttgaagtggtggcctaactacggctacactagaaggacagtatttggtatctgcgc
tctgctgaagccagttaccttcggaaaaagagttggtagctcttgatccggcaaacaaacca
ccgctggtagcggtggtttttttgtttgcaagcagcagattacgcgcagaaaaaaaggatct
caagaagatcctttgatcttttctacggggtctgacgctcagtggaacgaaaactcacgtta
agggattttggtcatgagattatcaaaaaggatcttcacctagatccttttaaattaaaaat
gaagttttaaatcaatctaaagtatatatgagtaaacttggtctgacagttaccaatgctta
atcagtgaggcacctatctcagcgatctgtctatttcgttcatccatagttgcctgactccc
cgtcgtgtagataactacgatacgggagggcttaccatctggccccagtgctgcaatgatac
cgcgagacccacgctcaccggctccagatttatcagcaataaaccagccagccggaagggcc
gagcgcagaagtggtcctgcaactttatccgcctccatccagtctattaattgttgccggga
agctagagtaagtagttcgccagttaatagtttgcgcaacgttgttgccattgctacaggca
tcgtggtgtcacgctcgtcgtttggtatggcttcattcagctccggttcccaacgatcaagg
cgagttacatgatcccccatgttgtgcaaaaaagcggttagctccttcggtcctccgatcgt
tgtcagaagtaagttggccgcagtgttatcactcatggttatggcagcactgcataattctc
ttactgtcatgccatccgtaagatgcttttctgtgactggtgagtactcaaccaagtcattc
tgagaatagtgtatgcggcgaccgagttgctcttgcccggcgtcaatacgggataataccgc
gccacatagcagaactttaaaagtgctcatcattggaaaacgttcttcggggcgaaaactct
caaggatcttaccgctgttgagatccagttcgatgtaacccactcgtgcacccaactgatct
tcagcatcttttactttcaccagcgtttctgggtgagcaaaaacaggaaggcaaaatgccgc
aaaaaagggaataagggcgacacggaaatgttgaatactcatactcttcctttttcaatatt
attgaagcatttatcagggttattgtctcatgagcggatacatatttgaatgtatttagaaa
aataaacaaataggggttccgcgcacatttccccgaaaagtgccacctgacgtctaagaaac
cattattatcatgacattaacctataaaaataggcgtatcacgaggccctttcgtctcgcgc
gtttcggtgatgacggtgaaaacctctgacacatgcagctcccggagacggtcacagcttgt
ctgtaagcggatgccgggagcagacaagcccgtcagggcgcgtcagcgggtgttggcgggtg

tcggggctggcttaactatgcggcatcagagcagattgtactgagagtgcaccatatgcggt
gtgaaataccgcacagatgcgtaaggagaaaataccgcatcaggaaattgtaaacgttaata
ttttgttaaaattcgcgttaaatttttgttaaatcagctcattttttaaccaataggccgaa
atcggcaaaatcccttataaatcaaaagaatagaccgagatagggttgagtgttgttccagt
ttggaacaagagtccactattaaagaacgtggactccaacgtcaaagggcgaaaaaccgtct
atcagggcgatggcccactacgtgaaccatcaccctaatcaagttttttggggtcgaggtgc
cgtaaagcactaaatcggaaccctaaagggagcccccgatttagagcttgacggggaaagcc
ggcgaacgtggcgagaaaggaagggaagaaagcgaaaggagcgggcgctagggcgctggcaa
gtgtagcggtcacgctgcgcgtaaccaccacacccgccgcgcttaatgcgccgctacagggc
gcgtcgcgccattcgccattcaggctacgcaactgttgggaagggcgatcggtgcgggcctc
ttcgctattacgccagctggctgca

SEQ ID NO 012

AAV2 5'ITR     1-143
CMV enhancer 169-721
MLC promoter 800 722-1491
SD/SA 1492-1669
5'miR flanking region 1762-1789
misiPLBh-shRNA 1790-1848
3'miR flanking region 1849-1893
SV40 poly A 1951-2203
AAV2 3'ITR 2210-2329

cctgcaggcagctgcgcgctcgctcgctcactgaggccgcccgggcaaagcccgggcgtcgg
gcgacctttggtcgcccggcctcagtgagcgagcgagcgcgcagagagggagtggccaactc
catcactaggggttcctgcggccgcagaattcggtaccgcggtggcggccgcttcgagctcg
cccgacattgattattgactagttattaatagtaatcaattacggggtcattagttcatagc
ccatatatggagttccgcgttacataacttacggtaaatggcccgcctggctgaccgcccaa
cgacccccgcccattgacgtcaataatgacgtatgttcccatagtaacgccaatagggactt
tccattgacgtcaatgggtggagtatttacggtaaactgcccacttggcagtacatcaagtg
tatcatatgccaagtacgccccctattgacgtcaatgacggtaaatggcccgcctggcatta
tgcccagtacatgaccttatgggactttcctacttggcagtacatctacgtattagtcatcg
ctattaccatggtgatgcggttttggcagtacatcaatgggcgtggatagcggtttgactca
cggggatttccaagtctccaccccattgacgtcaatgggagtttgttttggcaccaaaatca
acgggactttccaaaatgtcgtaacaactccgccccatgcggccgctctagccggaatcaaa
gctcaggctcctccctcttcctcctcctctgcccccctcctcctcctctgcccccctcttc
ctcctctgcccccctcttcttcctcctcctcttcctcctcccctcctcatctacctccttctc
ctcctcctcccccctcctcttcctcctctgcccccctcttcctcctcctcctcttcctcctcct
cttcctcctcccctcctcatctacctccttctcctcctcctcccccctcctcttcctcctctg
cccccctcttcctcctctgcccctcttcctcctcctcctcttcctcctctgcccccctcctccc
cctcctcttcctcttcctcctcccctcctcatctacctccttctcttcctcctcttcttcct
cctctttctcctcctcctccctctcctcttcctcctcctcttctttctcctcctcctcttcc
tccccctccccttcctgggttacttttccccattagacaatggcaggacccagagcacagag
catcgttcccaggccaggccccagccactgtctctttaaccttgaaggcattttgggtctc
acgtgtccacccaggcgggtgtcggactttgaacggctcttacttcagaagaacggcatggg
gtgggggggcttaggtggcctctgcctcacctacaactgccaaaagtggtcatggggttatt
tttaaccccagggaagaggtatttattgttccacagcaggggccggccagcaggctccttga
atttcgaggaactgaaaaaccagaaagttaactggtaagtttagtctttttgtcttttattt
caggtcccggatccggtggtggtgcaaatcaaagaactgctcctcagtggatgttgccttta
cttctaggcctgtacggaagtgttacttctgctctaaaagctgcggaattgtacccgcggcc

gcccccaattcgagctcgcccggggatcctctagagtcgacctgcagaagcttggtaccccg
gaggtagtgagtcgaccagtggatcctggaggcttgctgaaggctgtatgctgatacagatc
agcaagagacatgtttggccactgactgacatgtctctgatctgtatcaggacacaagg
cctgttactagcactcacatggaacaaatggcccagatctggccgcatcgagatcgacgcgt
gctagagctagaatcgataagctagcttctgtgccttctagttgccagccatctgttgtttg
cccctcccccgtgccttccttgaccctggaaggtgccactcccactgtcctttcctaataa
atgaggaaattgcatcgcattgtctgagtaggtgtcattctattctggggggtggggtgggg
caggacagcaagggggaggattgggaagacaatagcaggcatgctggggatgcggtgggctc
tatggcttctgaggcggaaagaaccagctggggtctagagtcccactccctctctgcgcgct
cgctcgctcactgaggccgggcgaccaaaggtcgcccgacgcccgggctttgcccgggcggc
ctcagtgagcgagcgagcgcgcagctgcctgcaggggcgcctgatgcggtattttctcctta
cgcatctgtgcggtatttcacaccgcatacgtcaaagcaaccatagtacgcgccctgtagcg
gcgcattaagcgcggcgggtgtggtggttacgcgcagcgtgaccgctacacttgccagcgcc
ctagcgcccgctcctttcgctttcttcccttcctttctcgccacgttcgccggctttccccg
tcaagctctaaatcgggggctccctttagggttccgatttagtgctttacggcacctcgacc
ccaaaaaacttgatttgggtgatggttcacgtagtgggccatcgccctgatagacggtttt
cgccctttgacgttggagtccacgttctttaatagtggactcttgttccaaactggaacaac
actcaaccctatctcggctattcttttgatttataagggattttgccgatttcggcctatt
ggttaaaaaatgagctgatttaacaaaaatttaacgcgaattttaacaaaatattaacgttt
acaattttatggtgcactctcagtacaatctgctctgatgccgcatagttaagccagccccg
acacccgccaacacccgctgacgcgccctgacgggcttgtctgctcccggcatccgcttaca
gacaagctgtgaccgtctccgggagctgcatgtgtcagaggttttcaccgtcatcaccgaaa
cgcgcgagacgaaagggcctcgtgatacgcctatttttataggttaatgtcatgataataat
ggtttcttagacgtcaggtggcacttttcggggaaatgtgcgcggaacccctatttgtttat
ttttctaaatacattcaaatatgtatccgctcatgagacaataaccctgataaatgcttcaa
taatattgaaaaaggaagagtatgagtattcaacatttccgtgtcgcccttattcccttttt
tgcggcattttgccttcctgtttttgctcacccagaaacgctggtgaaagtaaaagatgctg
aagatcagttgggtgcacgagtgggttacatcgaactggatctcaacagcggtaagatcctt
gagagttttcgccccgaagaacgttttccaatgatgagcacttttaaagttctgctatgtgg
cgcggtattatcccgtattgacgccgggcaagagcaactcggtcgccgcatacactattctc
agaatgacttggttgagtactcaccagtcacagaaaagcatcttacggatggcatgacagta
agagaattatgcagtgctgccataaccatgagtgataacactgcggccaacttacttctgac
aacgatcggaggaccgaaggagctaaccgcttttttgcacaacatgggggatcatgtaactc
gccttgatcgttgggaaccggagctgaatgaagccataccaaacgacgagcgtgacaccacg
atgcctgtagcaatggcaacaacgttgcgcaaactattaactggcgaactacttactctagc
ttcccggcaacaattaatagactggatggaggcggataaagttgcaggaccacttctgcgct
cggcccttccggctggctggtttattgctgataaatctggagccggtgagcgtgggtctcgc
ggtatcattgcagcactggggccagatggtaagccctcccgtatcgtagttatctacacgac
ggggagtcaggcaactatggatgaacgaaatagacagatcgctgagataggtgcctcactga
ttaagcattggtaactgtcagaccaagtttactcatatatactttagattgatttaaaactt
catttttaatttaaaaggatctaggtgaagatcctttttgataatctcatgaccaaaatccc
ttaacgtgagttttcgttccactgagcgtcagaccccgtagaaaagatcaaaggatcttctt
gagatcctttttttctgcgcgtaatctgctgcttgcaaacaaaaaaaccaccgctaccagcg
gtggtttgtttgccggatcaagagctaccaactctttttccgaaggtaactggcttcagcag
agcgcagataccaaatactgtccttctagtgtagccgtagttaggccaccacttcaagaact
ctgtagcaccgcctacatacctcgctctgctaatcctgttaccagtggctgctgccagtggc
gataagtcgtgtcttaccgggttggactcaagacgatagttaccggataaggcgcagcggtc
gggctgaacggggggttcgtgcacacagcccagcttggagcgaacgacctacaccgaactga
gatacctacagcgtgagctatgagaaagcgccacgcttcccgaagggagaaaggcggacagg
tatccggtaagcggcagggtcggaacaggagagcgcacgagggagcttccagggggaaacgc
ctggtatctttatagtcctgtcgggtttcgccacctctgacttgagcgtcgatttttgtgat

gctcgtcagggggcggagcctatggaaaaacgccagcaacgcggcctttttacggttcctg
gccttttgctggccttttgctcac

## Claims

1. RNA polynucleotide molecule comprising two sequence tracts that hybridize to each other by base-pairing in 5' to 3' direction of one sequence tract with the other in reverse orientation within the same molecule, whereby the sequence of one of the sequence tracts that hybridizes to the other is more than 80% similar to a sequence to a messenger RNA encoding phospholamban.

2. DNA polynucleotide sequence comprising
a. a first sequence element that when transcribed by an RNA polymerase renders a transcript capable of forming a partially self-complementary hairpin structure which can be processed by a eukaryotic cell to an siRNA product capable of degrading an mRNA in said cell, whereby the mRNA degraded by the siRNA product encodes phospholamban and
b. a promoter sequence operably linked to the first sequence element, said promoter sequence being operable by an RNA polymerase naturally occurring in a mammalian cell, whereby the promoter sequence is either a conditional promoter and / or a cardiomyocyte cell-specific promoter.

3. DNA polynucleotide sequence according to claim 2, **characterized in that** the eukaryotic cell is a mammalian cell.

4. DNA polynucleotide sequence according to claim 2 or 3, **characterized in that** the promoter sequence is an RNA polymerase II promoter.

5. DNA polynucleotide sequence according to claims 2 to 4, **characterized in that** the first sequence element further comprises sequence elements that facilitate the processing of the transcript by the splicing apparatus of the cell.

6. RNA polynucleotide molecule according to claim 1 or DNA polynucleotide sequence according to any of the preceding claims 2 to 5, comprising at least one of the sequences SEQ ID NO 001, SEQ ID NO 002 or SEQ ID NO 003.

7. DNA polynucleotide sequence according to claim 2 or 6, comprising at least one of the sequences SEQ ID NO 001, SEQ ID NO 002, SEQ ID NO 001, and at least one of the sequences SEQ ID NO 004 and SEQ ID NO 005 and at least one of the sequences SEQ ID NO 007, SEQ ID NO 008 or SEQ ID NO 009.

8. DNA polynucleotide sequence according to any of the above claims 2 to 7, further comprising a virus-derived DNA polynucleotide packaging sequence.

9. A DNA polynucleotide sequence according to claim 8, **characterized in that** the virus-derived DNA polynucleotide sequence is an adenovirus packaging sequence.

10. A DNA polynucleotide sequence according to claim 8, **characterized in that** the virus-derived DNA polynucleotide sequence is derived from an adeno- associated virus.

11. A DNA polynucleotide sequence according to claim 10, **characterised in that** the virus-derived DNA polynucleotide sequence is derived from adeno- associated virus type 9.

12. The DNA polynucleotide sequence of SEQ ID No 010 or SEQ ID NO 011 or SEQ ID NO 012.

13. A virus particle containing a polynucleotide sequence according to any of the preceding claims.

14. An isolated eukaryotic cell containing a polynucleotide sequence according to any of the claims 1 to 13.

15. An isolated eukaryotic cell according to claim 14, whereby the cell is a mammalian cell.

16. A composition for the treatment of cardiomyopathy comprising:
a. a polynucleotide sequence, a virus particle and / or an isolated mammalian cell according to any of the claims 1 to 16 and
b. a pharmaceutically acceptable excipient.

17. A composition for the treatment of cardiomyopathy comprising:
c. a polynucleotide sequence or a virus particle or an isolated mammalian cell according to any of the claims 1 to 16 and
d. a DNA polynucleotide sequence or a virus particle comprising an expression cassette comprising a second promoter sequence operable in a cardiomyocyte and a second sequence element encoding SERCA operably linked to said second promoter sequence, and
e. a pharmaceutically acceptable excipient.

18. The sequence of SEQ ID NO 009.
